# EUROPEAN PATENT SPECIFICATION

(11) **EP 3 452 281 B1**
(45) Date of publication and mention of the grant of the patent: **04.11.2020**
(21) Application number: 17732216.1
(22) Date of filing: 02.05.2017
(51) Int. Cl.: B32B 5/02, B32B 5/26, B32B 7/14, B32B 27/12

(54) **MULTILAYER MATERIAL COMPRISING AT LEAST A LAYER OF NON-WOVEN FABRIC**
MEHRSCHICHTIGES MATERIAL MIT MINDESTENS EINER SCHICHT AUS VLIESSTOFF
MATÉRIAU MULTICOUCHE COMPRENANT AU MOINS UNE COUCHE DE TISSU NON-TISSÉ

(30) Priority: 02.05.2016 IT UA20163073
(43) Date of publication of application: 13.03.2019
(73) Proprietor: Pantex International Spa A Socio Unico, 20060 Trezzano Rosa (IT)
(72) Inventor: ANGELI, Pietro, 20060 Trezzano Rosa (IT)
(74) Representative: Fiussello, Francesco
(86) International application number: PCT/IB2017/052538
(87) International publication number: WO 2017/191556

(56) References cited:
- EP-A1- 3 141 229
- WO-A1-2009/082277
- WO-A1-2015/146452
- WO-A1-2015/146717
- WO-A2-02/17843
- AU-B2- 736 963
- JP-A- 2009 000 512
- JP-A- 2015 043 895
- US-A1- 2008 026 178

## Description

### TECHNICAL FIELD

The present invention concerns a multilayer material comprising at least one layer of non-woven fabric.

### STATE OF THE ART

Multilayer structures are known suitable for use in absorbent sanitary articles.

By multilayer material in the context of the present invention we mean that the material comprises at least two distinct layers bonded together, for example, by means of lamination.

By absorbent sanitary article in the context of the present invention we mean all absorbent products such as, for example, disposable absorbent articles, sanitary towels, panty liners, interlabial devices, catamenial / medicinal / surgical tampons, nappies, incontinence pads, towels, medications, breastfeeding pads, underarm sweat guards, products for underwear, trousers and shorts, make-up remover wipes, mattress / bed / chair protector pads, absorbent swabs for animals and similar.

Below, by absorbent sanitary article we mean any one of the above-mentioned products.

Originally absorbent products were made of composite materials formed of a plurality of layers containing fabrics or fibres generally made of cotton which allowed body fluids to be absorbed.

Subsequently the structures were further developed, incorporating cellulose pulp which is able to absorb 5-6 times its weight. Nowadays, for example, the structures of sanitary articles and, in particular, of nappies, sanitary towels and incontinence pads, incorporate absorbent materials in gel such as polyacrylates in combination with cellulose fibres, for example, thus making it possible to market relatively fine multilayer structures.

Absorbent sanitary articles like children's nappies, sanitary towels or incontinence pads are typical applications.

For these purposes multilayer materials are used comprising a first layer in contact with the user's skin and called generally and here below topsheet, generally made of non-woven fabric. Below the topsheet, a further layer is optionally arranged adapted to acquisition and distribution of the liquid, followed by a layer of absorbent material and lastly a backsheet which must be waterproof.

The topsheet must be rapidly crossed by the liquids, but at the same time it must act as a barrier and avoid return of the liquids from the absorbent layer towards the user's skin, in particular it must avoid the surface in contact with the skin becoming wet and must maintain the feeling of dryness on the skin.

The more permeable the material composing the topsheet, the more efficiently it will allow liquids to pass through.

Furthermore, the topsheet is often required to be soft to the touch, since it is in contact with the user's skin, and also give the user a feeling of cushioning. It is furthermore desirable for the topsheet to have a resilience to dry and wet in all directions.

The thicker the layer of material, the more difficult it is for the liquid to permeate.

Said problem has been solved by perforating the topsheet to allow the liquids to pass through the layer and rapidly reach the liquid acquisition and distribution layer and the absorbent material below.

However, perforation of the topsheet to accelerate the passage of the liquids is obtained by crushing the layer of material forming the topsheet, reducing the thickness thereof and therefore reducing the softness of the topsheet.

To attempt to solve this problem, topsheets have been used consisting of special composite materials or different perforation methods have been used.

Generally, the topsheets are made of non-woven fabric.

Very thick materials produced with the so-called "air through bonded" technology, abbreviated to ATB, are frequently used as topsheets. The fibres that compose the strip of non-woven material are thermally bonded with a jet of hot air without contact with pressure cylinders, unlike what occurs with the more common carded thermal bonded materials.

In addition to being very expensive, the ATBs do not ensure a sufficient dryness effect, which is ideal for topsheets.

The commonest non-woven fabrics used as topsheets, although inexpensive, are no longer considered soft enough to the touch and therefore users are in search of alternatives that are more pleasant to wear.

To form topsheets having a greater cushioning effect, the non-woven fabric is formed by two rotating rollers, one female, i.e. with hollows, and one male, i.e. with reliefs corresponding to the hollows of the first roller so as to form 3D patterns or designs on the layer of final non-woven fabric, which give an impression of three-dimensionality and a volume or cushioning effect.

However, said counter-rotating rollers with male and female designs are extremely difficult and costly to produce. Furthermore, said rollers tend to become easily misaligned and therefore the resulting design on the topsheet is often inaccurate and does not allow the desired result to be obtained. Furthermore, the speed of the machine for winding the rollers is considerably slowed down to increase the precision of the design, but in this way the production costs of the material increase exponentially.

The need is therefore felt for a multilayer material comprising a topsheet for absorbent sanitary articles, in particular for children's nappies, incontinence pads or sanitary towels, able to solve the above-mentioned problems and which can also be produced at a lower cost.

WO0217843 discloses bonded composites, absorbent articles comprising such bonded composites, and processes for bonding thin-section elements. The bonded composite has first and second thin-section elements bonded to each other, at least in part by bond elements and at least inpart by adherent material. The adherent material is disposed between the first and second thin-section elements proximate and about the bond elements. The adherent material, at least in part, bonds the thin-section elements to each other at loci of the adherent material. The bond patterns are arranged and configured to preferentially direct stresses imposed on the bond pattern, inwardly into the interior of the bond pattern for distribution, dissipation, and termination

WO2015146452 discloses an absorbent article in which a top sheet having extruded protrusions and a second sheet are joined, wherein occurrence of wrinkles in the top sheet in an MD direction is prevented.

WO2009082277 discloses a seam joining together at least two web materials in an overlapped manner by ultrasonic welding, heat bonding or the like, in a bonding pattern comprising a first bonding pattern and at least one second bonding pattern extending in longitudinal direction along and adjacent at least one side edge of the overlapped portion. The bonded area of the second bonding pattern occupies more than 30 % of the combined bonded area of the first and second bonding patterns. The bonding elements of the first bonding pattern have a mean area that is at least 2 times the mean area of the bonding elements of the second bonding pattern. The contact area of the bonding pattern, as seen in transverse direction of the seam, is between 10 and 30% of the width (w) of the bonding pattern at any given point along the length of the bonding pattern.

WO2015146717 discloses a surface sheet (1A) for an absorbent article including large polygonal regions (BT) and small polygonal regions (ST) surrounded by a plurality of embossed parts. The embossed parts form top parts of the large polygonal regions (BT) and small polygonal regions (ST). A high projection) is formed inside each of the large polygonal regions (BT) and a low projection) is formed inside each of the small polygonal regions (ST).

AU736963 discloses a breathable elastic laminate formed by bonding a film including an elastic water vapor-soluble polymer to a neckable nonwoven web such that when the film is relaxed, the web is in a necked state. The breathable laminate is stretchable in a direction parallel to the narrowing or necking of the web. The laminate possesses excellent water vapor permeability but acts as a barrier to the passage of odor-causing chemicals including ammonia.

US2008026178 discloses a loop-forming nonwoven material used as a mechanical closure element, in particular for disposable sanitary articles such as various types of nappies, incontinence articles and pads. A first upper face of the nonwoven material has first larger non-bonded areas, which are located at a distance from one another in the form of islands. The first larger non-bonded areas of the nonwoven material are delimited by bonded contours and surrounded by second smaller non-bonded areas lying outside said delimitation and separating the larger areas from one another.

JP2009000512 discloses a surface sheet for an absorbable article, capable of rapidly performing the diffusion of a liquid and the transfer of the liquid in a thickness direction by allowing a configuration fiber to have density gradient in multiple directions.

JP2015043895 discloses a top sheet capable of preventing leak of a liquid by preventing the liquid from reaching end portions. The top sheet for absorbent articles includes a plurality of recessed joining points that are formed by partially fusing a plurality of superposed sheet members by embossment. The top sheet includes fused areas in which the joining points are formed at least parts of a prescribed direction, and unfused areas in which the joining points are not formed in the prescribed direction between the fused areas. At least parts of the unfused areas are bulged into a projecting shape equivalent to or more than unfused parts in the fused areas.

EP3141229 discloses a composite tape (T) comprising a first web (W1) and a second web (W2) welded together according to a welding pattern comprising a plurality of welding points arranged in arrays extending along a first direction (MD) and spaced apart in a second direction (CD), perpendicular to said first direction (MD), with adjacent arrays offset from each other in said first direction (MD), and with said welding points in each array arranged in groups spaced apart in said first direction (MD). The first web (W1) has a plurality of hollow protrusions alternating with welding points, both in said first direction (MD) and in said second direction (CD), and the second web (W2) has a plurality of ribs extending along said first direction (MD) and separated from each other by said arrays of welding points.

### SUMMARY OF THE INVENTION

The object of the invention is therefore to produce a multilayer material which is both soft, pleasant in contact with the skin and allows rapid passage of body fluids, but at the same time remains dry and can therefore be advantageously used as a topsheet for an absorbent sanitary article and has a resilience to dry and wet in all directions and an optimal cushioning effect.

According to the present invention the object is achieved by a multilayer material according to claim 1 and a process according to claim 6.

### BRIEF DESCRIPTION OF THE FIGURES

- Figure 1 is a photograph of a multilayer material comprising a layer of non-woven fabric according to the present invention;
- figure 2 is a schematic representation of a portion of the multilayer material of figure 1; and
- figure 3 is a section view of a projection of a cylinder adapted to form a semi-arcuate region along the axis III-III of the multilayer material of figure 2.

### DISCLOSURE OF THE INVENTION

In the context of the present invention, by layer we mean a mass of homogeneous material laid more or less uniformly over a surface.

According to the present invention it is advantageous to use the multilayer material as topsheet for an absorbent sanitary article.

In one aspect of the present invention, the multilayer material 1 comprises at least two layers. At least one of the two layers is a non-woven fabric 2.

More preferably the multilayer material comprises at least 3 layers.

Preferably the multilayer material comprises a second layer of non-woven fabric or at least one perforated layer, for example a perforated film.

Preferably the layer of non-woven fabric consists of a fibrous material in strip form, also known as web.

The process for producing a multilayer material 1 according to the present invention comprises the steps of unwinding a first layer of non-woven fabric from a first roller at a first rotation speed, unwinding a second layer from a second roller at a second rotation speed, passing the first layer and the second layer through a third and a fourth roller, keeping one against the other and rotating at a third rotation speed and at a fourth rotation speed respectively. Preferably the first rotation speed and the second rotation speed are different, the third roller is mainly smooth and the fourth roller has projections adapted to form connection regions 3 between the first layer 2 and the second layer of multilayer material. The projections are adapted to form connection regions 3 comprising a primary pattern consisting of at least one central region 5 and two non-rectilinear regions 6,7 arranged side-by-side but without contact between them and with the central region and forming overall an arc region 8 comprising a concave region 9.

In particular the first rotation speed is lower than the second rotation speed. In particular the fourth rotation speed is different from the third rotation speed.

Therefore, for the formation of the multilayer material, the two layers of identical or different materials are unwound at differentiated speeds starting from two rollers and then passed between two rotating counter rollers, one of which is smooth and the other provided with projections.

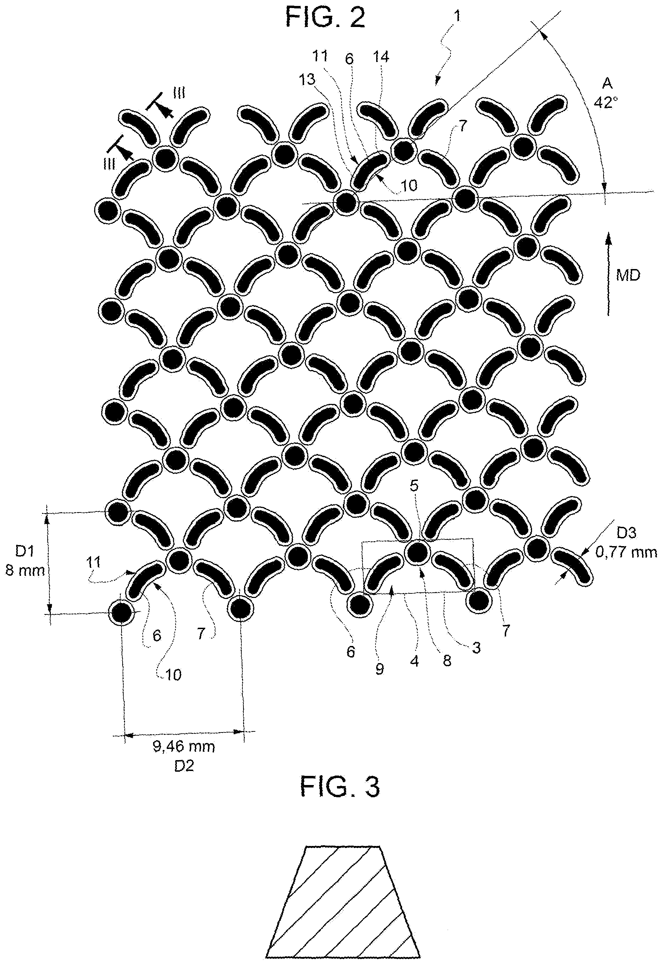

The projections have preferably in section, a truncated pyramid shape, as shown in Fig. 3.

The roller or cylinder from which a first strip of non-woven fabric is unwound rotates at a speed lower than the speed at which the second layer is unwound, which will form the multilayer material so that when the strip of non-woven fabric which will form the first layer passes on the contact line between the two cylinders, it is pre-tensioned.

By pre-tensioned we mean that the two unwinding speeds are different and therefore there is a differential tensioning between the two layers of material.

Said pre-tensioning ensures a better result during processing of the material and, in particular, during connection of the strip or layer of non-woven material to the nearby layer in the following step of the process.

In a following step, the two layers are passed between two rotating rollers to form connection regions between them and so as to form a design that follows a regular geometry, also called pattern, on at least one strip of non-woven fabric.

The two rollers are kept in contact against each other and preferably have different peripheral speeds and can connect the layers both by means of pressure and by increasing the temperature.

In particular, and more preferably, the smooth cylinder has a lower speed than the cylinder with projections to provide further improved results in terms of the volume or cushioning effect.

The layer 2 of non-woven fabric is therefore pre-tensioned in the machine direction before being combined or bonded to the following layer.

The machine direction or MD coincides with the direction in which the various layers of material are unwound to be processed.

In one aspect of the present invention, the projections on one of the rotating cylinders are adapted to form connection regions between the layer 2 of non-woven fabric and the layer below, whether formed of a layer of non-woven fabric or a perforated film.

The projections therefore have a form suited to producing connection regions adapted to form a pattern on the final layer of non-woven fabric.

The connection or contact or bonding regions or areas 3, also commonly called "bonding areas", on the non-woven fabric comprise non-rectilinear regions 6, 7, being semi-arcuate regions, for example similar to the shape of a comma or a kidney bean, like those schematically shown in Figure 2.

The semi-arcuate regions have a concave part and a convex part and the concave part faces towards the so-called machine direction.

More preferably the two semi-arcuate regions are arranged symmetrically around the central region.

Even more preferably, the line that joins the two terminal points of the concave part on the perimeter of the semi-arcuate region is arranged at an angle of between 30° and 60° with respect to the line perpendicular to the machine direction, even more preferably between 40° and 50°, for example 42° as shown in figure 2.

More preferably the connection areas are formed of a primary base pattern consisting of two semi-arcuate regions connected by a central region to form overall an arc which, during production of the material, has the concave part facing towards the machine direction or towards the two material unwinding rollers, i.e. the machine direction or MD.

By primary pattern we mean the simplest form which, when repeated, allows the overall pattern of the layer of non-woven fabric to be reproduced.

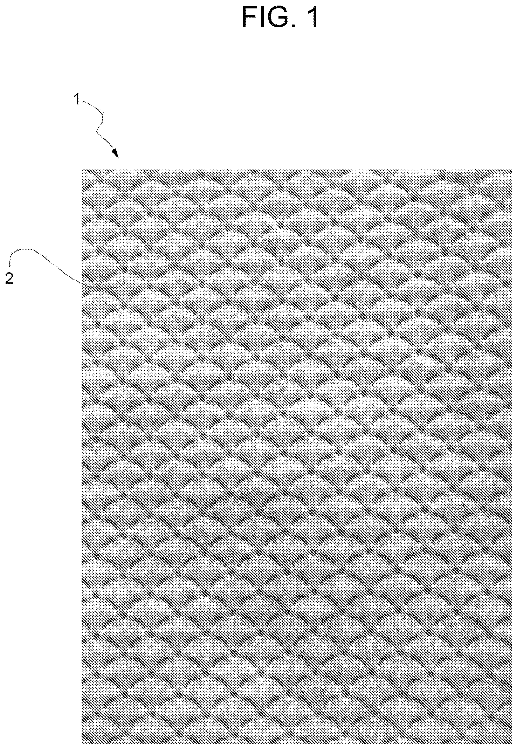

By overall pattern we mean the design which is formed overall on the layer of non-woven fabric by means of the connection areas as shown also graphically in figure 1 and schematically in figure 2.

Even more preferably the connection areas form overall a plurality of arc regions 8 each formed of a central region 5, with preferably circular or elliptical shape and arranged at the maximum height of the arc, also called "keystone", and two semi-arcuate regions 6, 7 so as to form a semicircular arc or a C turned by 90° with a concave part 9, which is preferably facing downwards or towards the machine direction or MD during production of the material.

The central region 5 is central or comprised between the semi-arcuate regions 6, 7 and with reference to the axis perpendicular to the machine direction MD in figure 2.

The central region 5 has a circular shape in the material as defined in claim 1 and a circular or an elliptical shape in the process as defined in claim 6.

The central region 5 and semi-arcuate regions 6, 7 represent the connection or contact regions with the layer below, i.e. those regions that have been formed by pressing against the surface of the smooth roller, if necessary with the aid of a higher temperature. The connection or union between the two layers can be obtained not only via the method described of the two rotating rollers and therefore by means of pressure and if necessary high temperature to form the embossed regions, but also via other methods known in the sector.

An example of a real layer of non-woven fabric is shown in figure 1, while figure 2 shows a diagram of the same product. Figure 3 shows a section of a projection on a roller adapted to form the semi-arcuate and central regions. The section is taken at a point adapted to form a semi-arcuate region at the axis III-III of figure 2.

In particular figure 1 shows the excellent cushioning effect on the finished product due to the shape of the connection areas which give the layer a three-dimensional effect and an effect of softness.

Preferably each arc region 8, which forms a primary pattern 4, is positioned alongside another arc region 8 to form an overall pattern in which each central region is surrounded by 4 semi-arcuate regions arranged in an X shape and the arc regions 8 follow one another regularly, more preferably symmetrically.

Considering the axis perpendicular to the machine direction, the entire non-rectilinear or semi-arcuate regions extend in an area preferably between 15° and 75°, more preferably between 30° and 60°, even more preferably between 40° and 50°. Preferably the connection areas 3 occupy a surface between 5 and 50% of the overall surface of the layer of non-woven fabric, more preferably between 10 and 30%.

Preferably the multilayer material 1 comprises two layers made of non-woven fabric. More preferably, both the non-woven fabrics have connection areas with the next layers having a pattern according to the present invention. Preferably the central points of the central regions are at a distance of between 2 and 20 mm, more preferably between 5 and 15 mm, even more preferably between 7 and 12 mm and they can be different in the direction perpendicular to that of the machine and in the machine direction. For example, they can be at a distance of 9,46 mm in the direction perpendicular to that of the machine and 8 in the machine direction.

Preferably the longest axis of the central regions is less than 5 mm, more preferably less than 2 mm.

Preferably the non-rectilinear or semi-arcuate regions have an overall length of less than 100 mm, more preferably less than 0.5 mm.

Preferably the non-rectilinear or semi-arcuate regions have a maximum width and a minimum width. Preferably the maximum width corresponds to the ends while the minimum width corresponds to the centre of the semi-arcuate region. Preferably the minimum width or the only width is less than 2 mm, more preferably less than 1 mm, for example it can be 0.77 mm.

From an examination of the characteristics of the multilayer material produced according to the present invention, the advantages it offers are evident.

It has been ascertained that the multilayer material according to the present invention allows an excellent three-dimensional effect to be obtained and ensures an adequate feeling of softness in particular when used as a topsheet in an absorbent article.

Furthermore, in aesthetic terms the resulting pattern is similar to the capitonne effect in armchairs and therefore creates an even greater impression of softness.

## Claims

1. A multilayer material (1) comprising at least one layer of non-woven fabric (2) and a second layer connected to said first layer by means of connection regions (3), said connection regions comprising a plurality of primary patterns (4), each primary pattern consisting of at least one central connection region (5) and two non-rectilinear connection regions (6,7) arranged alongside said central region, but without contact between each other and with said central region, **characterized in that** said central connection region has a circular shape and is central with respect to the two non-rectilinear connection regions (6, 7), said central connection region and said non-rectilinear connection regions form overall a semicircular arc region (8) comprising a concave region (9) and said central region is arranged at the maximum height of the arc and said two non-rectilinear connection regions (6,7) are semi-arcuate regions having a concave portion (10) and a convex portion (11) and said concave portions (10) of said semi-arcuate regions (6, 7) are all facing towards the concave regions (9).

2. The multilayer material (1) according to claim 1, **characterized in that** said second layer is a layer of non-woven fabric or a perforated film.

3. The multilayer material (1) according to claims 1 or 2, **characterized in that** it comprises a plurality of said arc regions (8) alongside one another in a regular succession of arc regions having the same dimensions.

4. The multilayer material according to any one of the preceding claims, **characterized in that** the line that joins the two terminal points (13, 14) of said concave regions (10) of said semi-arcuate regions (6, 7) is arranged at an angle ranging between 30° and 60° with respect to the line perpendicular to the unwinding direction of the material when wound on a roller or to the direction of the machine.

5. Absorbent sanitary article comprising a multilayer material according to any one of the claims from 1 to 4.

6. A process for the manufacture of a multilayer material (1) comprising the steps of:
- unwinding a first layer of non-woven fabric from a first roller at a first rotation speed
- unwinding a second layer from a second roller at a second rotation speed
- passing said first layer and said second layer through a third and a fourth roller held against each other and rotating at a third rotation speed and at a fourth rotation speed respectively, **characterized in that**:
- the first rotation speed and the second rotation speed are different
- said third roller is mainly smooth and said fourth roller has projections adapted to form connection regions (3) between said first layer (2) and said second layer of said multilayer material
- said projections are adapted to form connection regions (3) comprising a primary pattern consisting of at least one central region (5) and two non-rectilinear regions (6,7) arranged side-by-side but without contact between them and with said central region and forming overall a semicircular arc region (8) comprising a concave region (9); said central connection region has a circular or elliptical shape and is central with respect to the two non-rectilinear connection regions (6, 7); said central connection region is arranged at the maximum height of the arc and said two non-rectilinear connection regions (6,7) are semi-arcuate regions having a concave portion (10) and a convex portion (11) and said concave portions (10) of said semi-arcuate regions (6, 7) are all facing towards the concave regions (9).

7. The process according to claim 6 **characterized in that** said first rotation speed is lower than said second rotation speed.

8. The process according to claim 6 **characterized in that** said fourth rotation speed is different from said third rotation speed.

9. The process according to any of the claims from 6 to 8 **characterized in that** a multilayer material is produced according to any one of the claims from 2 to 5.

## Patentansprüche

1. Mehrschichtiges Material (1), das wenigstens eine Schicht aus einem Vliesstoff (2) und eine über Verbindungsbereiche (3) mit der ersten Schicht verbundene zweite Schicht umfasst, wobei die Verbindungsbereiche eine Vielzahl von primären Mustern (4) umfassen, wobei jedes primäre Muster aus wenigstens einem mittleren Verbindungsbereich (5) und zwei nicht-geradlinigen Verbindungsbereichen (6, 7), die entlang des mittleren Bereichs aber ohne Kontakt zueinander und zu dem mittleren Bereich angeordnet sind, besteht, **dadurch gekennzeichnet, dass** der mittlere Verbindungsbereich eine kreisrunde Form aufweist und mittig in Bezug auf die zwei nicht-geradlinigen Verbindungsbereiche (6, 7) angeordnet ist, wobei der mittlere Verbindungsbereich und die nicht-geradlinigen Verbindungsbereiche gemeinsam einen halbkreisförmigen Bogenbereich (8) einschließlich eines konkaven Bereichs (9) bilden und der mittlere Bereich an der maximalen Höhe des Bogens angeordnet ist und wobei die zwei nicht-geradlinigen Verbindungsbereiche (6, 7) halbbogenförmige Bereiche mit einem konkaven Teil (10) und einem konvexen Teil (11) sind und die konkaven Teile (10) der halbbogenförmigen Bereiche (6, 7) alle den konkaven Bereichen (9) zugewandt sind.

2. Mehrschichtiges Material (1) nach Anspruch 1, **dadurch gekennzeichnet, dass** die zweite Schicht eine Schicht aus einem Vliesstoff oder eine perforierte Folie ist.

3. Mehrschichtiges Material (1) nach Anspruch 1 oder 2, **dadurch gekennzeichnet, dass** es eine Vielzahl der Bogenbereiche (8) umfasst, die nebeneinander in einer regelmäßigen Folge von Bogenbereichen mit gleichen Dimensionen angeordnet sind.

4. Mehrschichtiges Material nach einem der vorstehenden Ansprüche, **dadurch gekennzeichnet, dass** die Linie, die die zwei Endpunkte (13, 14) der konkaven Bereiche (10) der halbbogenförmigen Bereiche (6, 7) verbindet, mit einem Winkel im Bereich von 30° bis 60° in Bezug auf die Linie senkrecht zu der Abwicklungsrichtung des auf eine Rolle gewickelten Materials oder auf die Richtung der Maschine angeordnet ist.

5. Absorbierender Hygieneartikel, der ein mehrschichtiges Material gemäß einem der Ansprüche 1 bis 4 umfasst.

6. Verfahren für die Herstellung eines mehrschichtigen Materials (1), das die folgenden Schritte umfasst:
- Abwickeln einer ersten Schicht eines Vliesstoffs von einer ersten Rolle mit einer ersten Drehgeschwindigkeit,
- Abwickeln einer zweiten Schicht von einer zweiten Rolle mit einer zweiten Drehgeschwindigkeit, und
- Führen der ersten Schicht und der zweiten Schicht zwischen einer dritten Rolle und einer vierten Rolle hindurch, die gegeneinander gehalten werden und sich jeweils mit einer dritten Drehgeschwindigkeit bzw. einer vierten Drehgeschwindigkeit drehen,
**dadurch gekennzeichnet, dass**:
- die erste Drehgeschwindigkeit und die zweite Drehgeschwindigkeit verschieden sind,
- die dritte Rolle vorwiegend glatt ist und die vierte Rolle Vorsprünge aufweist, die ausgebildet sind, um Verbindungsbereiche (3) zwischen der ersten Schicht (2) und der zweiten Schicht des mehrschichtigen Materials auszubilden, und
- die Vorsprünge ausgebildet sind zum Bilden von Verbindungsbereichen (3), die ein primäres Muster aufweisen, das aus wenigstens einem mittleren Bereich (5) und zwei nicht-geradlinigen Bereichen (6, 7) besteht, die nebeneinander, aber ohne Kontakt zueinander und zu dem mittleren Bereich angeordnet sind, und gemeinsam einen halbbogenförmigen Bereich (8) einschließlich eines konkaven Bereichs (9) bilden, wobei der mittlere Verbindungsbereich eine kreisförmige oder elliptische Form aufweist und mittig in Bezug auf die zwei nicht-geradlinigen Verbindungsbereiche (6, 7) angeordnet ist, wobei der mittige Verbindungsbereich an der maximalen Höhe des Bogens angeordnet ist und die zwei nicht-geradlinigen Verbindungsbereiche (6, 7) halbbogenförmige Bereiche mit einem konkaven Teil (10) und einem konvexen Teil (11) sind und wobei die konkaven Teile (10) der halbbogenförmigen Bereiche (6, 7) alle den konkaven Bereichen (9) zugewandt sind.

7. Verfahren nach Anspruch 6, **dadurch gekennzeichnet, dass** die erste Drehgeschwindigkeit niedriger als die zweite Drehgeschwindigkeit ist.

8. Verfahren nach Anspruch 6, **dadurch gekennzeichnet, dass** die vierte Drehgeschwindigkeit verschieden von der dritten Drehgeschwindigkeit ist.

9. Verfahren nach einem der Ansprüche 6 bis 8, **dadurch gekennzeichnet, dass** ein mehrschichtiges Material gemäß einem der Ansprüche 2 bis 5 erzeugt wird.

## Revendications

1. Matériau multicouche (1) comprenant au moins une couche de tissu non tissé (2) et une deuxième couche reliée à ladite première couche par le biais de zones de liaison (3), lesdites zones de liaison comprenant une pluralité de motifs principaux (4), chaque motif principal étant constitué d'au moins une zone de liaison centrale (5) et de deux zones de liaison non rectilignes (6, 7) agencées le long de ladite zone centrale, mais sans être en contact l'une avec l'autre ni avec ladite zone centrale, **caractérisé en ce que** ladite zone de liaison centrale présente une forme circulaire et est centrale par rapport aux deux zones de liaison non rectilignes (6, 7), ladite zone de liaison centrale et lesdites zones de liaison non rectilignes forment ensemble une zone d'arc semicirculaire (8) comprenant une zone concave (9) et ladite zone centrale est agencée au niveau de la hauteur maximale de l'arc et lesdites deux zones de liaison non rectilignes (6, 7) sont des zones semi-arquées présentant une partie concave (10) et une partie convexe (11) et lesdites parties concaves (10) desdites zones semi-arquées (6, 7) font toutes face aux zones concaves (9).

2. Matériau multicouche (1) selon la revendication 1, **caractérisé en ce que** ladite deuxième couche est une couche de tissu non tissé ou un film perforé.

3. Matériau multicouche (1) selon les revendications 1 ou 2, **caractérisé en ce qu'**il comprend une pluralité desdites zones d'arc (8) les unes le long des autres selon une succession régulière de zones d'arc ayant les mêmes dimensions.

4. Matériau multicouche selon l'une quelconque des revendications précédentes, **caractérisé en ce que** la droite qui réunit les deux points terminaux (13, 14) desdites zones concaves (10) desdites zones semi-arquées (6,7) est agencée à un angle allant de 30° à 60° par rapport à la droite perpendiculaire à la direction de déroulement du matériau lorsque celui-ci est enroulé sur un rouleau ou à la direction de la machine.

5. Article hygiénique absorbant comprenant un matériau multicouche selon l'une quelconque des revendications 1 à 4.

6. Procédé de fabrication d'un matériau multicouche (1) comprenant les étapes consistant à :
- dérouler une première couche de tissu non tissé à partir d'un premier rouleau à une première vitesse de rotation
- dérouler une deuxième couche à partir d'un deuxième rouleau à une deuxième vitesse de rotation
- faire passer ladite première couche et ladite deuxième couche entre un troisième et un quatrième rouleaux maintenus l'un contre l'autre et les faire tourner respectivement à une troisième vitesse de rotation et à une quatrième vitesse de rotation, **caractérisé en ce que** :
- la première vitesse de rotation et la deuxième vitesse de rotation sont différentes
- ledit troisième rouleau est principalement lisse et ledit quatrième rouleau présente des protubérances conçues pour former des zones de liaison (3) entre ladite première couche (2) et ladite deuxième couche dudit matériau multicouche
- lesdites protubérances sont conçues pour former des zones de liaison (3) comprenant un motif principal constitué d'au moins une zone centrale (5) et de deux zones non rectilignes (6, 7) agencées côte à côte mais sans être en contact l'une avec l'autre ni avec ladite zone centrale et formant ensemble une zone d'arc semicirculaire (8) comprenant une zone concave (9) ; ladite zone de liaison centrale présente une forme circulaire ou elliptique et est centrale par rapport aux deux zones de liaison non rectilignes (6, 7) ; ladite zone de liaison centrale est agencée au niveau de la hauteur maximale de l'arc et lesdites deux zones de liaison non rectilignes (6, 7) sont des zones semi-arquées présentant une partie concave (10) et une partie convexe (11) et lesdites parties concaves (10) desdites zones semi-arquées (6, 7) font toutes face aux zones concaves (9).

7. Procédé selon la revendication 6, **caractérisé en ce que** ladite première vitesse de rotation est inférieure à ladite deuxième vitesse de rotation.

8. Procédé selon la revendication 6, **caractérisé en ce que** ladite quatrième vitesse de rotation est différente de ladite troisième vitesse de rotation.

9. Procédé selon l'une quelconque des revendications 6 à 8, **caractérisé en ce qu'**un matériau multicouche est produit selon l'une quelconque des revendications 2 à 5.
